# EUROPÄISCHE PATENTSCHRIFT

(11) **EP 1 255 721 B1**
(45) Veröffentlichungstag und Bekanntmachung des Hinweises auf die Patenterteilung: **01.10.2008**
(21) Anmeldenummer: 01915166.1
(22) Anmeldetag: 26.01.2001
(51) Int. Cl.: C07C 45/50

(54) **VERFAHREN ZUR HYDROFORMYLIERUNG VON OLEFINEN MIT 20 BIS 400 KOHLENSTOFFATOMEN**
METHOD FOR HYDROFORMYLATING OLEFINS HAVING BETWEEN 20 AND 400 CARBON ATOMS
PROCEDE D'HYDROFORMYLATION D'OLEFINES AYANT ENTRE 20 ET 400 ATOMES DE CARBONE

(30) Priorität: 28.01.2000 DE 10003871
(43) Veröffentlichungstag der Anmeldung: 13.11.2002
(73) Patentinhaber: BASF SE, 67056 Ludwigshafen (DE)
(72) Erfinder: SCHWAHN, Harald, 69168 Wiesloch (DE); BORCHERT, Gerhard, 67069 Ludwigshafen (DE); DIEHL, Klaus, 67454 Hassloch (DE); GRENACHER, Armin, Volker, 67112 Mutterstadt (DE); SAUER, Friedrich, 67271 Obersülzen (DE)
(74) Vertreter: Kinzebach, Werner
(86) Internationale Anmeldenummer: PCT/EP2001/000853
(87) Internationale Veröffentlichungsnummer: WO 2001/055068

(56) Entgegenhaltungen:
- WO-A-98/12235
- DRAXLER J ET AL: "AUSLEGUNGSKRITERIEN FUER ELEKTROSTATISCHE EMULSIONSSPALTANLAGEN*" CHEMIE. INGENIEUR. TECHNIK,DE,VERLAG CHEMIE GMBH. WEINHEIM, Bd. 62, Nr. 7, 1. Juli 1990 (1990-07-01), Seiten 525-530, XP000171965 ISSN: 0009-286X in der Anmeldung erwähnt

## Beschreibung

Die vorliegende Erfindung betrifft ein Verfahren zur Hydroformylierung von Olefinen mit 20 bis 400 Kohlenstoffatomen durch Umsetzung der Olefine mit Synthesegas in Gegenwart eines Kobaltcarbonyl-Katalysators.

Die Hydroformylierung bzw. Oxo-Synthese ist ein seit Jahrzehnten großtechnisch ausgeübtes Verfahren, bei dem aus Olefinen durch Umsetzung mit Gemischen von Kohlenmonoxid und Wasserstoff in Gegenwart von Carbonylkomplexen von Metallen der VIII. Nebengruppe des Periodensystems, insbesondere denjenigen des Kobalts oder Rhodiums, die um ein Kohlenstoffatom verlängerten Aldehyde hergestellt werden (vgl. dazu die Monographie "New Syntheses with Carbon Monoxide", J. Falbe (Hrsg.), Springer Verlag 1980.

Zur Hydroformylierung längerkettiger Olefine wird gegenwärtig praktisch ausschließlich Kobalt als katalytisch wirksames Metall eingesetzt. Die bekannten Verfahrensvarianten der kobaltkatalysierten Hydroformylierung unterscheiden sich vor allem in der Art und Weise der Abtrennung des homogen im Reaktionsgemisch gelösten Katalysators von den Reaktionsprodukten. Aus Gründen der Wirtschaftlichkeit und zur Befreiung des Hydroformylierungsprodukts vom Katalysator muss dieser möglichst vollständig abgetrennt und in die Synthesestufe zurückgeführt werden. Eine elegante Möglichkeit zur Katalysatorabtrennung ist die Heterogenisierung des homogen gelösten Katalysators in einer mit dem Hydroformylierungsprodukt nicht mischbaren flüssigen Phase.

Dazu wird üblicherweise gemäß den Angaben der DE-A-2404855 das Reaktionsgemisch mit molekularem Sauerstoff in Gegenwart von wässriger Säure behandelt. Dabei wird das Kobalt von der Oxidationsstufe -1 nach +2 oxidiert und kann sodann durch Extraktion mit der wässrigen Lösung entfernt werden. Die Abtrennung des wässrigen Extraktes erfolgt z. B. durch Dekantieren in einem Phasentrenngefäß oder in anderen dafür geeigneten Einrichtungen.

Bei der Hydroformylierung kurzkettiger Olefine beträgt die in der organischen Phase verbleibende Restmenge an Kobalt üblicherweise weniger als etwa 2 ppm. Mit steigender Kettenlänge der erzeugten Aldehyd/Alkohol-Gemische, d. h. bei Kohlenstoffzahlen von mehr als 12, nehmen die grenzflächenaktiven Eigenschaften der Reaktionsprodukte zu. Dies hat zur Folge, dass die im Entkobaltungsschritt zunächst entstehende feindisperse flüssig-flüssig-Gas-Dispersion stabilisiert wird und die Tropfen-Tropfen-Koaleszenz bzw. Tropfen-Grenzflächen-Koaleszenz gehemmt wird. Erst bei längeren Verweilzeiten zerfällt die Emulsion weitgehend in die beiden flüssigen, in sich homogenen Phasen.

Die DE-AS-1285997 und die US-A-3488184 beschreiben die Entfernung von Kobalt(II)-salzen aus den Reaktionsprodukten der Oxo-Synthese mit Hilfe von Kationenaustauschern. Nachteilig für einen großtechnischen Dauereinsatz ist das Quellverhalten vieler Austauscherharze gegenüber aldehydhaltigem Reaktionsprodukt sowie die aufwendige Regenerierung der Austauscherharze.

Eine Anwendung der Hydroformylierungsreaktion unter Verwendung eines Kobaltkatalysators, die von besonderem Interesse ist, stellt gemäß EP-A-244616 die Hydroformylierung von Polybutenen oder Polyisobutenen zu Polybutyl- bzw. Polyisobutylaldehyden, -alkoholen oder -estern dar.

Bedingt durch die hohe Viskosität und die oberflächenaktiven Eigenschaften der Oxo-Produkte der Poly(iso)butene ist eine wirksame Entfernung des eingesetzten Kobaltkatalysators nur schwer zu erreichen. Bei der oxidativen Zerstörung der im Reaktoraustrag enthaltenen Kobaltcarbonyle in Gegenwart saurer wässriger Lösung bilden sich äußerst stabile Wasser-in-Öl-Emulsionen. Die Phasentrennung aufgrund der Dichtedifferenz erfordert sehr lange Verweilzeiten, wodurch eine wirtschaftliche Abtrennung im Erdschwerefeld nicht möglich ist.

Die WO 98/12235 beschreibt den kombinierten Einsatz von polymeren Emulsionsspaltern und koaleszenzfördernden Apparaten, um die Phasentrennung von Reaktionsausträgen der Formylierung von Olefinen mit 12 bis 100 C-Atomen zu beschleunigen. Während es mit Hilfe dieser Vorgehensweise gelingt, den Kobaltrestgehalt in den Hydroformylierungsausträgen der C₁₂-C₁₈-Olefine unter 1 ppm abzusenken, verbleibt in den Oxo-Produkten der (Iso)Butenoligomere mit 20 Kohlenstoffatomen oder mehr ein Kobaltrestgehalt von 6 bis 9 ppm. Diese Kobaltrestmengen können sich störend bei der Weiterverarbeitung der Hydroformylierungsprodukte auswirken. Sowohl bei der destillativen Aufarbeitung als auch bei chemischen Umsetzungen in Gegenwart von Wasserstoff, wie der Hydrierung oder der hydrierenden Aminierung, können sich feste Beläge von Kobaltsalzen oder metallischem Kobalt in den Apparaturen abscheiden, die den Stoff-und/oder Wärmetransport beeinträchtigen. Die Abscheidungen müssen in periodischen Abständen mechanisch oder chemisch, z. B. durch Auflösen in Salpetersäure, entfernt werden. Diese erforderlichen Maßnahmen sind umständlich und beeinträchtigen die Wirtschaftlichkeit der Weiterverarbeitungsschritte.

Die WO 98/12235 weist auf die Möglichkeit hin, die Abtrennung der feindispergierten Restwassermenge von der organischen Phase mittels elektrostatischer Koalesziervorrichtungen anstelle mechanischer Koalesziereinrichtungen vorzunehmen.

Derartige elektrostatische Koalesziervorrichtungen werden bereits bei der Erdölgewinnung zur Abtrennung salzhaltigen Wassers, das aus den Lagerstätten stammt und im Rohöl emulgiert vorliegt, verwendet, vgl. z. B. "Encyclopedia of Chemical Processing and Design", Vol. 17, S. 223, New York 1983; und Chem.-Ing.-Techn. 62 (1990), Nr. 7, S. 525. In der erstgenannten Literaturstelle wird auf S. 224 ausgeführt: "Alle [elektrischen] Entsalzer erfordern die Zugabe von Waschwasser, üblicherweise im Bereich von 4 bis 8 Vol-%, bezogen auf die Roheinsatzmenge."

Es hat sich gezeigt, dass beim Versuch, die nach wässriger Aufarbeitung eines Reaktionsaustrages der Hydroformylierung von Poly(iso)butenen und anschließender Phasentrennung erhaltene, z. B. noch bis zu 5 Gew.-% Wasser enthaltende organische Phase in einer elektrostatischen Koalesziervorrichtung zu trennen, Kurzschlussströme und die Abscheidung von metallischem Kobalt beobachtet werden, was nachteilig ist. Dieses Phänomen beruht vermutlich auf der hohen elektrischen Leitfähigkeit der wässrigen Phase aufgrund des Gehalts an gelösten Kobalt(II)-salzen sowie der Neigung der emulgierten Wassertröpfchen zur Ausbildung von perlschnurartigen Aggregaten in Feldrichtung und der vergleichsweise leichten Reduzierbarkeit der Kobalt(II)-salze zu metallischem Kobalt. Diese Probleme erschienen systemimanent, und eine naheliegende Lösung war nicht greifbar.

Der vorliegenden Erfindung liegt die Aufgabe zugrunde, Reaktionsausträge aus der kobaltkatalysierten Hydroformylierung von Olefinen mit 20 bis 400 Kohlenstoffatomen auf Kobaltrestgehalte von 2 ppm oder weniger, insbesondere 1 ppm oder weniger, zu reinigen und dafür ein effizientes und im großtechnischen Dauerbetrieb zuverlässiges Verfahren anzugeben.

Erfindungsgemäß wird diese Aufgabe gelöst durch ein Verfahren zur Hydroformylierung von Olefinen mit 20 bis 400 Kohlenstoffatomen durch Umsetzung der Olefine mit Synthesegas in Gegenwart eines Kobaltcarbonyl-Katalysators und Rückgewinnen des Kobaltkatalysators durch Extraktion des Reaktionsaustrages mit einer wässrigen sauren Lösung in Gegenwart von Sauerstoff und Trennung der organischen und wässrigen Phase, das dadurch gekennzeichnet ist, dass man
(a) unter Nutzung von Massekräften die wässrige Phase soweit von der organischen Phase abtrennt, dass der Anteil an in der organischen Phase dispergierter wässriger Phase 2 Gew.-% oder weniger, bezogen auf die organische Phase, beträgt,
(b) die in Schritt (a) erhaltene organische Phase zur Koaleszenz der restlichen dispergierten wässrigen Phase einem elektrischen Feld aussetzt.

Es ist kritisch für das erfindungsgemäße Verfahren, dass der Gehalt an dispergierter Wasserphase auf 2 Gew.-% oder weniger, vorzugsweise 1 Gew.-% oder weniger, insbesondere 0,5 Gew.-% oder weniger, verringert wird, bevor die organische Phase einer Elektrokoalesziervorrichtung zugeleitet wird. Der so von der Hauptmenge der wässrigen Phase befreiten organischen Phase wird kein Wasser zugesetzt. Dieser Befund ist überraschend, denn er wendet sich gegen die ausdrückliche Anweisung im Stand der Technik (vgl. oben "Encyclopedia of Chemical Processing and Design", Vol. 17), wonach bei der Entfernung von Salzlösungen aus Öl mittels Elektrokoaleszenz zur zu spaltenden Rohemulsion 4 bis 8 Vol-% Waschwasser zugesetzt werden sollen.

Die Hydroformylierung erfolgt in an sich bekannten Weise. Sie erfolgt geeigneterweise bei Drucken von 100 bis 400 bar und bei einer Temperatur von 100 bis 200 °C. Das Synthesegas enthält Kohlenmonoxid und Wasserstoff im Allgemeinen im Verhältnis von 1:10 bis 10:1. Der Kobaltcarbonyl-Katalysator wird zweckmäßigerweise in situ im Hydroformylierungsreaktor aus einer wässrigen Kobalt(II)-salzlösung, z. B. Kobalt(II)-formiat oder -acetatlösung, gebildet.

Als zu hydroformylierende Olefine kommen solche mit 20 bis 400 Kohlenstoffatome in Betracht, insbesondere Polyalkene, d. h. Oligomere oder Polymere von C₂-C₆-Alkenen, wobei die Oligomere oder Polymere olefinisch ungesättigt sind. Insbesondere kommen Polyisobutene, vorzugsweise Polyisobutene mit überwiegend endständiger Doppelbindung, wie sie z. B. in der US-A-5,286,823 offenbart sind, in Betracht.

Gewünschtenfalls können zur Viskositätserniedrigung inerte organische Verdünnungsmittel, wie gesättigte aliphatische Kohlenwasserstoffe oder aromatische Kohlenwasserstoffe, mitverwendet werden.

Der Reaktionsaustrag aus der Hydroformylierung wird geeigneterweise nach verlassen der Reaktionszone auf Mitteldruck, in der Regel 10 bis 30 bar, entspannt und in die Entkobaltungsstufe geleitet. In der Entkobaltungsstufe wird der Reaktionsaustrag in Gegenwart von wässriger, schwach saurer Lösung, z. B. mit einem pH-Wert von 2 bis 6, mit Luft oder Sauerstoff bei Temperaturen von vorzugsweise 90 bis 130 °C von Kobaltcarbonylkomplexen befreit. Die Entkobaltung kann gewünschtenfalls in einem mit Füllkörpern, wie z. B. Raschig-Ringen, gefüllten Druckbehälter durchgeführt werden, in dem eine möglichst hohe Phasenaustauschfläche erzeugt wird.

Aus dem erhaltenen Gemisch von wässriger Phase und organischer Phase wird erfindungsgemäß die wässrige Phase unter Nutzung von Massekräften soweit abgetrennt, dass der Anteil an dispergierter wässriger Phase 2 Gew.-% oder weniger, vorzugsweise 1 Gew.-% oder weniger, insbesondere 0,5 Gew.-% oder weniger, beträgt.

Zu den Massekräften nutzenden Abtrenntechniken zählen Absetzenlassen, Zentrifugieren und mechanische Koaleszenzstufen sowie Kombinationen davon. Am_ meisten bevorzugt ist eine Kombination von (i) Absetzenlassen und/oder Zentrifugieren, und (ii) einer oder mehreren mechanischen Koaleszenzstufen. In der Regel führt man zunächst durch Absetzenlassen und/oder Zentrifugieren eine weitgehende Abtrennung der wässrigen Phase unter Erhalt einer Feinemulsion herbei, die sich nicht mehr spontan entmischt und meist noch mehr als 2 bis 5 Gew.-% wässrige Phase enthält.

Zum Absetzenlassen kann das Gemisch aus wässriger und organischer Phase in eine Beruhigungszone geleitet und aufgetrennt werden. Dies geschieht zweckmäßigerweise in einem liegenden, kontinuierlich betriebenen Phasentrenngefäß, das mit geringer Strömungsgeschwindigkeit durchflossen wird. Bedingt durch den Dichteunterschied der Phasen trennt sich die Emulsion im Erdschwerefeld, so dass beide Phasen in zusammenhängender Form und weitgehend fremdphasenfrei übereinandergeschichtet vorliegen. Man erhält die wässrige Phase praktisch frei von organischer Phase, so dass die Kobaltsalzlösung ohne weitere Aufarbeitung in die Entkobaltungsstufe zurückgeführt werden kann.

Bevor die als Feinemulsion anfallende organische Phase der Koaleszenz im elektrischen Feld zugeführt werden kann, muss der Restgehalt an dispergierter wässriger Phase auf 2 Gew.-% oder weniger verringert werden. Hierzu benutzt man mit Vorteil eine oder mehrere mechanische Koaleszenzstufen mit integrierter oder nachgeschalteter Phasentrenneinrichtung. Geeignet sind im Allgemeinen Abscheider mit Koaleszenzeinbauten, wie Füllkörpern, Koaleszenzflächen oder feinporigen Elementen.

Bei den Koaleszenzflächeneinbauten handelt es sich in der Regel um Plattenpakete mit gewellten oder schräggestellten Flächen, an denen sich dispergierte Tröpfchen anlagern und zunächst einen Film ausbilden. Wenn dieser Film die Einzelplatte umschließt und dick genug ist, dann bilden sich große Tropfen der dispergierten Phase an der Plattenkante aus und fallen nach unten. Sie bilden dann eine Schicht im Abscheider, die mechanisch leicht abzutrennen ist.

Bei feinporigen Einbauten zwingt die innere Struktur der Elemente die feindispergierten Tropfen zum Kontakt mit der inneren Oberfläche, die dann einen Film bilden und als vereinigte größere Tropfen die hohle Struktur der feinporigen Elemente verlassen.

Als Füllkörper sind die üblicherweise bei der Destillation verwendeten Füllkörper geeignet. Vorzugsweise leitet man die Feindispersion von oben nach unten durch eine Füllkörperschüttung. Durch Benetzung der großen Füllkörperoberfläche kommt es zur Oberflächenkoaleszenz und gleichzeitig durch Tropfenbewegung zur Tropfen-Tropfen-Koaleszenz. In einer zweckmäßigen Ausführungsform wird eine vertikal angeordnete Füllkörperkolonne verwendet, wobei die Füllkörper aus einem Material bestehen, das durch die disperse wässrige Phase benetzt wird, und die Füllkörperschüttung durch die organische Phase geflutet ist. Bevorzugt werden Füllkörperkolonnen verwendet, die mit Füllkörpern aus Metall, z. B. Metallringen, gefüllt sind. Die sich bildenden großen Tropfen der wässrigen Phase scheiden sich rasch ab und können als Unterphase abgezogen werden. Das Hydroformylierungsprodukt wird oberhalb der Phasentrennschicht abgenommen.

Nach der (letzten) mechanischen Koaleszenzstufe enthält die organische Phase 2 Gew.-% oder weniger, vorzugsweise 1 Gew.-% oder weniger, insbesondere 0,5 Gew.-% oder weniger, z. B. 0,05 bis 0,3 Gew.-% dispergierte wässrige Phase, in Form einer Feinstemulsion.

Zweckmäßigerweise hält man im Hinblick auf eine vorteilhafte Viskosität des Hydroformylierungsproduktes bei der Massekräfte nutzenden Abtrennung der wässrigen Phase, insbesondere in der bevorzugten mechanischen Koaleszenzstufe, eine Temperatur von 50 bis 120 °C ein. Die Einhaltung einer Temperatur im angegebenen Bereich ist auch in der Elektrokoaleszenzstufe vorteilhaft.

Bei der Phasentrennung, insbesondere bei der Massekräfte nutzenden Abtrennung der wässrigen Phase, werden mit Vorteil Emulsionsspalter mitverwendet. Als Emulsionsspalter kommen insbesondere alkoxylierte Verbindungen in Betracht, wie sie üblicherweise in der Erdölindustrie zur Abtrennung des salzhaltigen Wassers Verwendung finden. Diese sind z. B.
(a) mit Propylenoxid und gegebenenfalls zusätzlich Ethylenoxid alkoxylierte Oligo- und Polyamine und -imine sowie
(b) alkoxylierte Alkylphenolformaldehydharze und
(c) Ethylenoxid/Propylenoxid-Blockcopolymere sowie
(d) deren polymere Acrylsäureester,

wie sie in der DE-A-2227546 und der DE-A-2435713 (a); DE-A-2013820 (b); DE-A-1545215 (c) und der DE-A-4326772 (d) beschrieben sind.

Besonders bevorzugt ist die Verwendung eines Emulsionsspalters, der durch Umsetzung von Polyethylenimin mit einem Molekulargewicht von 10 000 bis 50 000 mit solchen Mengen Propylenoxid und gegebenenfalls zusätzlich Ethylenoxid erhalten wird, dass der Gehalt an Alkoxyeinheiten 90 bis 99 Gew.-% beträgt.

Die Aufwandmenge an Emulsionsspaltern, die zur Erzielung des gewünschten Effekts zudosiert wird, beträgt etwa 0,1 bis 100 g/t des eingesetzten organischen Materials, vorzugsweise 2 bis 20 g/t.

Bevorzugt wird der Emulsionsspalter in verdünnter Form kontinuierlich zugegeben. Die Verdünnung mit einem inerten Lösungsmittel, z. B. o-Xylol, erleichtert die Handhabung sowie die Dosierung der geringen benötigten Menge. Die Zugabe erfolgt zweckmäßigerweise nach der Entkobaltung, vorzugsweise zusammen mit der Zugabe der wässrigen Extraktionslösung und der Luft bei der Entspannung, wodurch der Emulsionsspalter wirkungsvoll eingemischt wird.

Die organische Phase, die unter Nutzung von Massekräften von der Hauptmenge der wässrigen Phase befreit worden ist, wird anschließend zur Tropfen-Tropfen-Koaleszenz der dispergierten Tröpfchen der restlichen wässrigen Phase einem elektrischen Feld ausgesetzt. Die koaleszierte wässrige Phase kann dann in einer integrierten oder nachgeschalteten Phasentrenneinrichtung abgetrennt werden. Zur Koaleszenz im elektrischen Feld kann prinzipiell jede Anordnung aus zwei Elektroden verwendet werden, zwischen die die Feinstemulsion aus organischer Phase und restlicher dispergierter wässriger Phase eingebracht werden kann. Übliche Bauformen sind
(a) sogenannte Erdölspalter, die über eine Metallelektrode verfügen, wobei die bereits koaleszierte wässrige Phase als zweite Elektrode wirkt, so dass das elektrische Feld zwischen Metallelektrode und Grenzschicht wirkt,
(b) Ringspalter, die zwei konzentrische Elektroden aufweisen, wobei die innere Elektrode meist eine Stabelektrode ist;
(c) Plattenspalter, bei denen die Elektroden als parallele Platten ausgebildet sind.

Besonders bewährt haben sich Ringspalter, insbesondere solche, bei denen die äußere Elektrode in einem Abstand von etwa 100 mm um eine als Stabelelektrode ausgebildete innere Elektrode angeordnet ist.

Man kann mit Gleichspannung oder Wechselspannung arbeiten. Geeignete Gleichspannungen betragen 5 bis 40 kV, vorzugsweise 10 bis 40 kV. Geeignete Wechselspannungen betragen 0,5 bis 20 kV, vorzugsweise 3 bis 5 kV, bei Frequenzen von 50 bis 20 000 Hz.

Das erzeugte elektrische Feld ist vorzugsweise inhomogen. Ferner ist bevorzugt, dass die elektrische Feldrichtung senkrecht zur Schwerkraft wirkt.

Bei der Emulsionsspaltung im elektrischen Feld wird eine Erhöhung der Koaleszenzrate durch eine elektrisch induzierte Bewegung der Tropfen verursacht. Im inhomogenen elektrischen Feld bewegen sich die Tropfen nicht zu den Elektroden, sondern immer in Richtung höherer Feldstärke. Die durch Koaleszenz entstehenden größeren Tröpfchen können sich dann, der Schwerkraft folgend, schneller absetzen.

Durch die Behandlung im elektrischen Feld kann die wässrige Phase bis auf Werte entsprechend der Löslichkeit der wässrigen Phase im Hydroformylierungsprodukt abgeschieden werden. Der Kobaltrestgehalt lässt sich auf diese Weise bis auf Werte von 2 ppm oder weniger, meist 1 ppm oder weniger, vorzugsweise 0,8 ppm oder weniger, senken.

Eine vorteilhafte Ausführungsform des erfindungsgemäßen Verfahrens wird anhand der beigefügten schematischen Zeichnung im Einzelnen erläutert:

Über die Leitungen (1) bis (3) werden dem Hydroformylierungsreaktor (4) Olefin (1) und Synthesegase (Oxogas) (2) sowie eine wässrige Kobaltsalzlösung (3) zugeführt. Im Reaktor (4) findet bei üblichen Hydroformylierungsbedingungen die Umsetzung mit Oxogas zu den sauerstoffhaltigen Verbindungen statt. Der den aktiven Katalysator in Form des Kobaltcarbonylwasserstoffes enthaltende Austrag wird über die Leitung (5) der Entkobaltung (6) zugeführt und dort mit Luft über Leitung (7) und einer wässrigen sauren Kobaltsalzlösung (8) behandelt. Dabei wechselt das Kobalt die Oxidationsstufe von -1 nach +2 und wird in der sauren wässrigen Phase als Kobaltsalz gelöst. Unmittelbar nach der Entkobaltung wird über die Leitung (9) ein Emulsionsspalter zugegeben und der rohe Reaktionsaustrag über Leitung (10) in ein Phasentrenngefäß (11) geleitet. Hier trennen sich die Gasphase und die beiden Flüssigphasen weitgehend. Über die Leitung (12) werden die nichtumgesetzten Anteile der Luft sowie aus der Synthesestufe mitgeführte Anteile an CO und H₂ abgeleitet.

Nach der Phasentrennung (11) wird die geringe Menge der wässrigen Phase enthaltende organische Phase über die Leitung (13) in eine mechanische Koaleszenzstufe (14), vorzugsweise in eine Füllkörperkolonne, die mit Metallfüllkörpern, z. B. Pallringen, gefüllt sind, geleitet. Im nachgeschalteten Phasentrenngefäß (16), in die die organische Phase über die Leitung (15) gelangt, werden die durch die Koaleszenz vergrößerten Tröpfchen der wässrigen Phase abgeschieden.

Der mechanischen Koaleszenzstufe ist über Leitung (17) eine elektrische Koaleszenzstufe (18) nachgeschaltet, in der die feindispergierte wässrige Phase einem elektrischen Feld ausgesetzt wird. Die durch die Koaleszenz vergrößerten Tröpfchen der wässrigen Phase werden vollständig abgeschieden, und man erhält ein praktisch kobaltfreies Produkt, das in üblicher Weise aufgearbeitet werden kann.

Die in den Stufen (11), (16) und (18) abgeschiedene wässrige Phase (8) wird zum Teil in die Entkobaltungsstufe (6) und zum Teil in den Hydroformylierungsreaktor (4) zurückgeführt.

Das erfindungsgemäße Verfahren wird durch die nachfolgenden Beispiele näher erläutert.

### Beispiele

### Beispiel 1 (Vergleich, nicht erfindungsgemäß)

In einen Hydroformylierungsreaktor wurden stündlich 3 660 kg einer Mischung aus 1 940 kg Polyisobutenen (M_{N} etwa 1 000) und 1 720 kg einer C₁₀-C₁₄-Paraffinkohlenwasserstofffraktion sowie 300 kg einer wässrig sauren Kobaltformiatlösung, die 1,3 Gew.-% Kobalt enthält, eingeleitet. Das Gemisch wurde bei 183 bis 185 °C mit einem CO/H₂-Gemisch (40 Vol-% CO; 59,5 Vol-% H₂; 0,5 Vol-% Inerte) umgesetzt. Zur Aufrechterhaltung eines Gesamtdruckes von 275 bar wurde dem Reaktor druckgeregelt ständig Frischgas der vorgenannten Zusammensetzung zugeführt.

Nach Durchlauf der Reaktionszone wurde das Produkt in eine Entkobaltungsstufe unter Absenkung des Druckes auf etwa 20 bar entspannt. Gleichzeitig wurden in die Entkobaltungszone stündlich 2 600 kg Kobaltformiatlösung der oben genannten Zusammensetzung sowie 17 kg Luft geleitet. Unmittelbar nach dem Ausgang der Entkobaltungsstufe wurde ein Emulsionsspalter als verdünnte Lösung so zugegeben, dass die Konzentration 12 g/t Reaktoraustrag beträgt. Als Emulsionsspalter diente ein mit Propylenoxid modifiziertes Polyethylenimin (Molgewicht des zur Herstellung verwendeten Polyethylenimins etwa 20 000; Gehalt an Propoxy-Einheiten 99 Gew.-%).

Die vereinigten Ströme aus der Entkobaltungsstufe gelangten nach Passieren einer Mischstrecke in ein Phasentrenngefäß, aus dem pro Stunde 200 kg Entspannungsgas in ein Sammelsystem abgeleitet werden. Die Flüssigphasen trennten sich weitgehend voneinander. Die wässrige Kobaltformiatlösung war praktisch frei von organischen Anteilen und wird teilweise in die Entkobaltungsstufe und teilweise in den Reaktor zurückgeführt.

Die organische Phase enthielt noch etwa 0,7 Gew.-% emulgierte Kobaltformiatlösung. Zu deren Abreicherung wurde das Reaktionsprodukt auf 80 °C abgekühlt und von oben nach unten durch eine senkrecht stehende Füllkörperkolonne (Länge 6,5 m, Durchmesser 0,7 m) geleitet. Unterhalb der Schüttung setzte sich eine wässrige Kobaltformiatlösung ab, die in den Prozess zurückgeführt wurde, während die organische Phase oberhalb der Grenzschicht abgenommen wurde. Die leicht getrübte organische Phase enthielt noch 0,14 Gew.-% Wasser sowie 8 ppm Kobalt. Die Vorschrift zur Kobaltbestimmung ist nachstehend angegeben.

### Beispiel 2 (erfindungsgemäß)

Von der nach Beispiel 1 aus der Füllkörperkolonne abgezogenen, leicht getrübten organischen Phase wurden im Bypass stündlich 330 kg abgezweigt und einer scale-up-fähigen, senkrecht stehenden Zelle eines industrieüblichen Metercell^{®}-Abscheiders der Petrolite Corp., Houston, Texas, mit konzentrisch angeordneter, unisolierter Stabelelektrode bei 70 °C zugeführt. Der Abscheider stand unter einem Druck von 1,1 bar, die Querschnittsbelastung betrug etwa 13,5 m³/m² x h. Die Zelle wurde mit einer Gleichspannung von 40 kV betrieben, die Stromaufnahme lag unter 5 mA. Die aus dem Metercell^{®} -Abscheider ablaufende organische Phase war im Gegensatz zum Hauptstrom wasserklar und wies nur noch einen Restwassergehalt entsprechend der Löslichkeit bei 70°C von 0,06 Gew.-% auf. Der Kobaltgehalt war auf unter 1 ppm gefallen. Am Boden der Zelle schieden sich stündlich ca. 250 ml wässrige Phase ab, die 1,3 Gew.-% Kobaltsalz (ber. Co) enthielten.

### Beispiel 3 (erfindungsgemäß)

Von der nach Beispiel 1 aus der Füllkörperkolonne abgezogenen, leicht trüben organischen Phase wurden 100 ml in die Zelle eines temperierbaren, elektrostatischen Laborabscheiders eingefüllt. Der Abscheider war mit zwei konzentrischen Elektroden ausgerüstet, wobei die innere Elektrode als Metallstabelelektrode (Durchmesser 8 mm) ausgeführt war und die äußere Elektrode durch eine im Ringraum befindliche Elektrolytlösung (Salzlösung) gebildet wurde, welche über die Glaswand (Glasrohrinnendurchmesser 20 mm) isoliert war. Über einen weiteren Ringraum wurde durch zirkulierendes Heizmittel der Abscheider auf 70 °C temperiert. Da der Abscheider in Glas ausgeführt war, konnte der Trennvorgang sehr gut beobachtet werden. Durch Anlegung von 4 kV Wechselspannung (50 Hz) erfolgte innerhalb von 10 Sekunden eine vollständige Abtrennung der dispersen kobaltsalzhaltigen Wasserphase. Im Gegensatz zur ursprünglichen Probe war die organische Phase nun wasserklar und wies bei 70 °C einen Restwassergehalt von 0,06 Gew.-% auf, welcher der Löslichkeit entspricht. Der Kobaltgehalt der klaren organischen Phase lag unter 1 ppm. Am Boden der Zelle hat sich die rosafarbene, kobaltsalzhaltige Wasserphase abgetrennt.

Vorschrift zur massenspektrometrischen Kobalt-Bestimmung

Etwa 2 g der Probe werden einem automatisierten Aufschlussverfahren unterzogen (zunächst Vercrackung mit 10 ml Schwefelsäure bei etwa 320 °C; danach Zerstörung des Rückstandes mit 8 ml Mischsäure bei etwa 160 °C), bei dem die organischen Anteile der Probe durch heiße, konzentrierte Mineralsäuren vercrackt und vollständig oxidiert werden. Nach Abrauchen der Mineralsäuren bleibt das Kobalt in salzsaurer Lösung zurück. Die Aufschlusslösung wird auf 25 ml eingestellt. In dieser Lösung wird der Kobaltgehalt durch Massenspektrometrie mit induktiv gekoppeltem Plasma (ICP-MS) bestimmt.

### Folgende Reagenzien werden verwendet:

| Bi-destilliertes oder | deionisiertes Wasser |
|---|---|
| Schwefelsäure, β (H₂SO₄) | ca. 1,84 g/ml |
| Salzsäure, c(HCl) | ca. 5 mol/l |
| Salpetersäure, β(HNO₃) | ca. 1,41 g/ml |
| Perchlorsäure, β(HClO₄) | ca. 1,67 g/ml |

| | |
|---|---|
| Mischsäure: Eine Mischung von Salpetersäure, Schwefelsäure, Perchlorsäure im Volumenverhältnis 2:1:1. | |

| Geräte: | | |
|---|---|---|
| ICP-MS-Spektrometer, Messbedingungen: | z. B. Fa. Perkin Elmer Zerstäuber: Crossflow | "Elan 5000" |
| | Generator: 1 050 W | |
| | Messzeit: 1,5 s | |
| | Masse: 59 | |

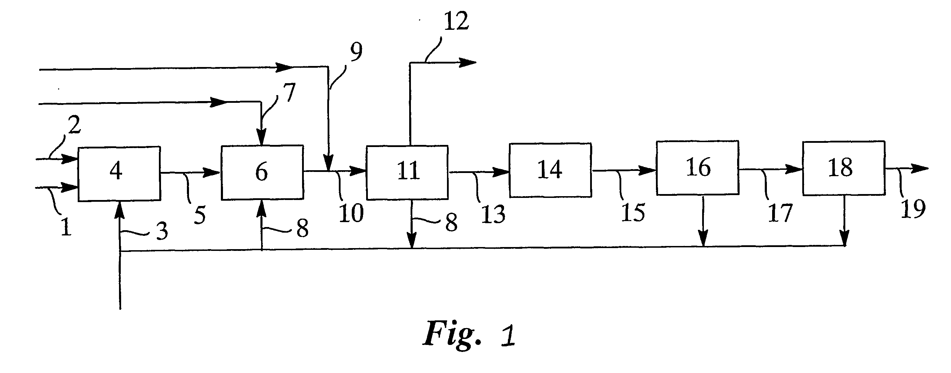

## Patentansprüche

1. Verfahren zur Hydroformylierung von Olefinen mit 20 bis 400 Kohlenstoffatomen durch Umsetzung der Olefine mit Synthesegas in Gegenwart eines Kobaltcarbonyl-Katalysators und Rückgewinnen des Kobaltkatalysators durch Extraktion des Reaktionsaustrages mit einer wässrigen sauren Lösung in Gegenwart von Sauerstoff und Trennung der organischen und wässrigen Phase, **dadurch gekennzeichnet, dass** man
(a) durch eine Kombination von (i) Absetzenlassen und/oder Zentrifugieren, und (ii) einer oder mehreren mechanischen Koaleszenzstufen die wässrige Phase soweit von der organischen Phase abtrennt, dass der Anteil an in der organischen Phase dispergierter wässriger Phase 0,5 Gew.-% oder weniger, bezogen auf die organische Phase, beträgt; und
(b) die in Schritt (a) erhaltene organische Phase zur Koaleszenz der restlichen dispergierten wässrigen Phase einem elektrischen Feld aussetzt.

2. Verfahren nach Anspruch 1, **dadurch gekennzeichnet, dass** man ein inhomogenes elektrisches Feld verwendet.

3. Verfahren nach Anspruch 1 oder 2, **dadurch gekennzeichnet, dass** man ein Gleichspannungsfeld einer Spannung von 5 bis 40 kV verwendet.

4. Verfahren nach einem der vorhergehenden Ansprüche, **dadurch gekennzeichnet, dass** man ein Wechselspannungsfeld einer Spannung von 0,5 bis 20 kV und einer Frequenz von 50 bis 20 000 Hz verwendet.

5. Verfahren nach einem der vorhergehenden Ansprüche, **dadurch gekennzeichnet, dass** die Phasentrennung in Gegenwart eines Emulsionsspalters erfolgt.

6. Verfahren nach Anspruch 5, **dadurch gekennzeichnet, dass** man als Emulsionsspalter ein mit Propylenoxid und/oder Ethylenoxid alkoxiliertes Polyethylenimin verwendet.

7. Verfahren nach einem der Ansprüche 1 bis 6, **dadurch gekennzeichnet, dass** man als mechanische Koaleszenzstufe eine vertikal angeordnete Füllkörperkolonne verwendet, wobei die Füllkörper aus einem Material bestehen, das durch die disperse wässrige Phase benetzt wird und die Füllkörperschüttung durch die organische Phase geflutet ist.

8. Verfahren nach einem der vorhergehenden Ansprüche, **dadurch gekennzeichnet, dass** man in den Schritten (a) und/oder (b) eine Temperatur zwischen 50 und 120 °C einhält.

9. Verfahren nach einem der vorhergehenden Ansprüche, **dadurch gekennzeichnet, dass** man als Olefin Polyisobuten einsetzt.

## Claims

1. A process for the hydroformylation of olefins having from 20 to 400 carbon atoms by reaction of the olefins with synthesis gas in the presence of a cobalt carbonyl catalyst and recovery of the cobalt catalyst by extraction of the reaction product with an aqueous acidic solution in the presence of oxygen and separation of the organic and aqueous phases, wherein
(a) the aqueous phase is separated from the organic phase by a combination of (i) settling and/or centrifugation, and (ii) one or more mechanical coalescence stages to the extent that the proportion of aqueous phase dispersed in the organic phase is 0.5% by weight or less, based on the organic phase; and
(b) the organic phase obtained in step (a) is exposed to an electric field to coalesce the remaining dispersed aqueous phase.

2. The process according to claim 1, wherein an inhomogeneous electric field is used.

3. The process according to claim 1 or 2, wherein a DC field having a voltage of from 5 to 40 kV is used.

4. The process according to any of the preceding claims, wherein an AC field having a voltage of from 0.5 to 20 kV and a frequency of from 50 to 20,000 Hz is used.

5. The process according to any of the preceding claims, wherein the phase separation is carried out in the presence of an emulsion breaker.

6. The process according to claim 5, wherein the emulsion breaker used is a polyethylenimine alkoxylated with propylene oxide and/or ethylene oxide.

7. The process according to any of claims 1 to 6, wherein the mechanical coalescence stage used is a vertical packed column in which the packing elements are made of a material which is wetted by the disperse aqueous phase and the bed of packing is flooded by the organic phase.

8. The process according to any of the preceding claims, wherein a temperature of from 50 to 120°C is maintained in steps (a) and/or (b).

9. The process according to any of the preceding claims, wherein the olefin used is polyisobutene.

## Revendications

1. Procédé en vue de l'hydroformylation d'oléfines ayant de 20 à 400 atomes de carbone par réaction des oléfines avec le gaz de synthèse en présence d'un catalyseur cobalt-carbonyle et par récupération du catalyseur cobalt par extraction du produit de réaction à l'aide d'une solution acide aqueuse en présence d'oxygène et par séparation des phases organique et aqueuse, **caractérisé en ce que**
(a) l'on sépare, par une combinaison (i) d'une mise au repos pour sédimentation et/ou d'une centrifugation et (ii) d'une ou de plusieurs étapes de coalescence mécaniques, la phase aqueuse de la phase organique dans une mesure telle que la proportion de phase aqueuse dispersée dans la phase organique soit de 0,5 % en poids ou moins, par rapport à la phase organique; et
(b)l'on expose la phase organique obtenue dans l'étape (a), en vue de la coalescence de la phase aqueuse dispersée résiduelle, à un champ électrique.

2. Procédé selon la revendication 1, **caractérisé en ce que** l'on utilise un champ électrique non-homogène.

3. Procédé selon la revendication 1 ou 2, **caractérisé en ce que** l'on utilise un champ à tension constante d'une tension de 5 à 40 kV.

4. Procédé selon l'une quelconque des revendications précédentes, **caractérisé en ce que** l'on utilise un champ à tension alternative d'une tension de 0,5 à 20 kV et d'une fréquence de 50 à 20 000 Hz.

5. Procédé selon l'une quelconque des revendications précédentes, **caractérisé en ce que** la séparation de phases se fait en présence d'un séparateur d'émulsions.

6. Procédé selon la revendication 5, **caractérisé en ce que** l'on utilise, en tant que séparateur d'émulsions, un polyéthylène-imine alcoxylé à l'oxyde de propylène et/ou à l'oxyde d'éthylène.

7. Procédé selon l'une quelconque des revendications 1 à 6, **caractérisé en ce que** l'on utilise, en tant qu'étape de coalescence mécanique, une colonne de corps de remplissage disposée verticalement, le corps de remplissage se composant d'un matériau qui est mouillé par la phase aqueuse dispersée et la masse en vrac de corps de remplissage étant noyée par la phase organique.

8. Procédé selon l'une quelconque des revendications précédentes, **caractérisé en ce que** l'on maintient, dans les étapes (a) et/ou (b), une température comprise entre 50 et 120°C.

9. Procédé selon l'une quelconque des revendications précédentes, **caractérisé en ce que** l'on utilise, en tant qu'oléfine, un polyisobutène.
